# EUROPEAN PATENT SPECIFICATION

(11) **EP 3 988 133 B1**
(45) Date of publication and mention of the grant of the patent: **02.09.2026**
(21) Application number: 20020483.2
(22) Date of filing: 20.10.2020
(51) Int. Cl.: A61L 9/04, A61L 9/12, B05B 7/00

(54) **AROMA DIFFUSER**
AROMA-DIFFUSOR
DIFFUSEUR D'ARÔME

(43) Date of publication of application: 27.04.2022
(73) Proprietor: Aroma General Sp. z o.o., 30-529 Kraków (PL)
(72) Inventor: Tryznowski, Mariusz, 04-838 Warszawa (PL); Przetacznik, Pawel, 41-205 Sosnowiec (PL); Grudziaz, Lukasz, 03-995 Warszawa (PL); Zolek-Tryznowska, Zuzanna, 04-838 Warszawa (PL)
(74) Representative: Radlowski, Jakub

(56) References cited:
- FR-A1- 3 005 267
- JP-A- 2011 011 746
- US-A- 5 503 139
- US-A1- 2018 369 507

## Description

The invention relates to a diffuser used in fragrance dispensers, in particular in automatic dispensers.

Different types of automatic dispensers of aromas, in particular of essential oils, are widely used. In the case of essential oil dispensers, aromas are emitted with the use of ultrasounds, a substance heater or gas atomiser (Venturi tube), etc. The aroma emitting module is activated automatically for example by a timer or motion detector.

A diffuser with which the fragrance is emitted into the atmosphere plays an important role in aroma dispensers.

In the aroma dispensers used today, there is a problem related to different densities of fragrances, most often dissolved in various oils. Some fragrances are easily dissolved in light fractions, whereas other fragrances require oils with greater density. In devices of a relatively simple structure such as the aroma dispenser, it leads to a significantly diversified intensity of emission with the same power of the atomiser. For aromas with small density the emission may be extensive, whereas "heavy" aromas partially condensate before they leave the dispenser. Consequently, for some aromas, the fragrance drips down the dispenser casing. In extreme cases, the aroma emitting nozzles get clogged.

The known technical solutions describe a construction of fragrance diffusion modules.

The American patent application No US 2020237950 entitled *"Heating and atomizing* aroma *diffuser*" presents the diffuser which consists of a cover with an inlet hole, atomiser equipped with a heating element and a hose supplying the aroma liquid wrapped around the heating component, as well as an aroma container and air flow generator with an inlet and a control module. The control module is connected electrically to the heating module and the air flow generator. The fragrance is emitted when exposed to high temperature, and then in a gaseous form it is blown out into the atmosphere while passing the chamber where it condenses partially. In this solution the fragrance emitting nozzle is in the form of a channel which widens conically at the outlet, and its diameter at the point of contact with the cover is greater than the diameter of the outlet hole. In the preferable variant, the outlet hole has a narrowing conical channel from the inside located inside the outlet channel. The steam pressure is reduced in this way and the fragrance condenses partially in the outlet channel. This solution allows to a certain, although limited, extent to prevent the problem signalled at the beginning, related to the condensation of heavy volatile fractions of fragrances at the outlet or on the dispenser casing.

The Chinese patent application No CN110772657 entitled *"Intelligent* aroma *diffuser facilitating water adding and drainage, and using method thereof'* presents the aroma dispenser in which the ultrasonic atomiser is located inside the atomising chamber, and this chamber is located inside the dispenser chamber and it is connected to the internal space with a narrow channel through which the fragrance escapes in a gaseous form. From the space inside the casing the fragrance escapes into the atmosphere. Some of the fragrance which does not penetrate into the outlet channel undergoes condensation and remains in the atomising chamber. In the lower part of the atomising chamber there is a drain hose which can be used to drain the container. In this application, the gaseous fragrance released into the inside of the casing may condense in the casing or may clog the outlet hole or flow through this hole to the outside of the casing.

The international patent application No WO2020019638 entitled *"Aroma diffuser"* presents the diffuser casing consisting of a round base threaded on the outer side and a round cover, the flange of which is threaded on the inner side. The cover and the base are aligned and connected with the use of the thread, so that when one of the components is rotated, the distance between the surface of the base and the cover changes. In the central part of the cover there is an outlet hole. There is a round separating plate between the base and the cover. It has holes arranged close to its edge. If the cover is twisted to the maximum level, its upper surface surrounding the outlet hole adjoins the holes in the separating plates thus totally blocking the fragrance flow. Twisting the cover off causes the space between its upper surface and the holes in the separating plate to open smoothly, increasing the aroma flow. This solution does not describe the construction of the dispenser in detail and does not allow to eliminate the technical problem described at the beginning.

The French patent application No FR 3005267 entitled "Monobloc essential oil diffuser refill bottle" presents the diffuser refill bottle of essential oil comprising of container of oil and a diffusion head comprising a spray chamber for producing oil droplets and being arranged to initially direct the stream of oil droplets created within it towards the inside of the container. The spray chamber comprises a compressed air inlet, an oil inlet and a mixing zone. The diffusion head has also a diffusion chamber arranged to brake the oil droplets into micro-droplets and diffuse said micro-droplets into the ambient air in the form of an oil vapour. The diffusion head also comprises a threaded portion cooperating by screwing with a threaded portion of the container. The bottle is characterized in that said diffusion head comprises elements capable of preventing unscrewing between the threaded portion of the diffusion head and the threaded portion of the container. In this solution the problem to be solved is to prevent disconnection of the diffusion head form the container and it's to be achieved by a threaded connection between the diffusion head and the container. The Japanese patent No. JP 2011 011746 entitled "Volatilization vessel of volatile liquid" presents a volatilization vessel comprising a vessel body accommodated in a case body with its upper being opened and with a liquid suction wick being hanged and fixed therein. There is a cap attached to the vessel body and provided with a press-breakable and easily-cuttable part on its top part. There is also a case lid which can be fitted with a different depth at the horizontally turning position an the predetermined angle with respect to the case body, and a volatilization plate which is pierced in and fixed to a lid fixing part. The liquid suction wick and the volatilization plate communicate with each other when the case lid is fitted from the shallow fitting position to the deep fitting position in a horizontally turning state at the predetermined angle to open an outside air introducing passage for communicating with the inside and the outside of the vessel body through a cut-through portion of the easily-cuttable part. The Japanese invention provides a volatilization vessel which is capable of easily executing the operation from a sealed state to a usable state of a vessel body before the use in a very easy manner compared with a conventional one, and reliably and easily forming an outside air introducing passage for communicating with the inside and the outside of a vessel body after the unsealing, and extremely convenient after the unsealing.

The invention relates to a diffuser according to claim 1 consisting of a casing, inside which there are structural components that force appropriate flow of the gaseous fragrance and discharge of condensate. The operating principle of the diffuser consists in the generation of aerosol in the atomising chamber and then the separation of larger particles of aerosol on discs in the cascade separation chamber, where the aerosol flow path goes up through the above-mentioned cascade system. The diffuser construction as presented enables the release of the particles of size < 10 µm, whereas larger particles return to the diffusion process after condensation in the atomising chamber and the separation chamber.

The diffuser has preferably the shape similar to an elliptical cylinder. In the dispenser base there is a discharge channel connecting the atomising chamber of the diffuser with the fragrance container. The atomising chamber is combined with the separating discs located above.

A spray nozzle, which is connected to the fragrance container by a supply channel, is routed to the atomising chamber. This nozzle feeds the compressed gas into the atomising chamber by sucking in the fragrance which expands in the atomising chamber and becomes aerosol. In the atomising chamber, the particles partially lose their momentum and undergo condensation into the fractions with the largest diameter of drops. The spray nozzle is preferably positioned in an intersection point of a longer axis of symmetry of oval or ellipse of atomising chamber (in a top view) and its wall. The diameter of the spray nozzle tip is preferably from 0.4 to 0.9 mm.

In the upper part of the separating system, there is a cover with an outlet hole through which the fragrance escapes into the atmosphere. Under the cover there is an upper separating chamber.

Between the atomising chamber located in the lower part of the dispenser and the upper separating chamber there is a lower separating chamber. In this chamber there is an upper separating disc with at least one upper ring (preferably two or more upper rings) formed on its bottom surface. Below there is a lower separating disc with at least one lower ring (preferably two or more lower rings) formed on its upper surface.

The lower and upper rings have preferably a round or elliptical shape and adjoin the lower and upper disc respectively, running substantially lengthwise in relation to the disc perimeter.

As a rule, the rings are connected to the discs perpendicularly, although they may be positioned at a certain angle, which does not violate the principle of the solution. If the rings are round in shape, the radius of every ring (both upper and lower rings) is different. This way the upper and lower rings gear, moving through the rings of the opposite disc. The upper and lower discs and the rings may also have an oval, elliptical or similar shape. Should this be the case, the above-mentioned rule shall apply accordingly, which means that for rings with an oval, elliptical or similar shape, the dimensions of individual upper and lower rings are different so that the rings move through the rings of the opposite discs and gear.

The lower rings connected to the lower disc are not in contact with the upper disc, and the upper rings connected to the upper disc are not in contact with the lower disc. Consequently, slots are created between the edges of the rings and the opposite discs through which the aerosol with gaseous fragrance penetrates.

In the upper part of the atomising chamber there might be a cover of the chamber with a hole through which a sleeve enters, forming a passage between the atomising chamber and the lower separation chamber. The hole or passage is made near the middle of the lower separation disc, on the inner side of the smallest lower ring. The sleeve has preferably a size and shape to fit perfectly in the hole in the cover of the atomising chamber. In this way, the lower disc is attached centrically in the cover of the atomising chamber.

Between the lower disc and the atomising chamber there is a free space forming the condensate chamber. The bottom part of the condensate chamber is preferably inclined towards the inside of the diffuser, at an angle that will enable free flow of oil. Preferably at an angle α 10 to 45°. In the lower disc, there are small holes between the rings with a diameter of 0.1 to 3 mm, through which the fragrance condensed between the lower and upper rings drips downs into the condensate chamber. Preferably between each pair of adjacent lower rings there are from 4 to 32 holes distributed evenly around the axis of symmetry of the diffuser.

The diameter of the lower disc is slightly lower than the inner diameter of the flange of the diffuser base. Therefore, between the lower disc and the casing there is a middle annular slot through which the fragrance, condensed before it penetrated the through holes and reached the upper separation chamber, drips down into the condensate chamber and flows outside the area of the outer ring with the largest radius. The size of the middle annular slot enables the flow of condensate, but it is small enough to prevent the penetration of the aerosol from the condensate chamber upwards. Preferably the width of the first annular slot is 0.5 to 0.8 mm.

Between the condensate chamber and the atomising chamber there is a lower annular slot through which the condensate drips down with the help of gravity to the atomising chamber and then through the discharge channel into the fragrance container. In this case also the width of the slot is preferably 0.5 to 0.8 mm.

Preferably in the largest upper ring there are circumferential holes on the perimeter forming gaps in the ring path. The circumferential holes may also have a different shape, e.g. ring perforations. In this variant the largest upper ring acts also as a spacer keeping the appropriate distance between the upper and the lower disc, so that the ring slots located between the rings and the opposite discs have the set width. Consequently, between this ring and the lower disc there is no slot through which the aerosol could penetrate, and this function is assumed by the circumferential holes through which the aerosol flows into the outer part of the lower separation chamber. In this outer part of the lower separation chamber, on the plane of the upper disc there is a least one through hole, and preferably several through holes, connecting the lower separation chamber with the upper separation chamber. The aerosol penetrates these holes and flows upwards. Preferably the through holes are positioned in the immediate vicinity of the circumferential holes. The through holes may have a round, oval or elliptical shape.

In the upper separation chamber, a certain amount of the gaseous fragrance with particles of size >10 µm undergoes condensation before the aerosol leaves the diffuser. To enable the flow of the oil downwards, towards the fragrance container, an upper annular slot was located between the outer edge of the upper disc and the casing. The condensate created in the upper separation chamber drips down through it into the lower separation chamber and then through the middle annular slot into the condensate chamber. Preferably the width of the upper annular slot is 0.5 to 0.8 mm.

In a preferable variant of the solution, the atomising chamber is elongated and is oval or elliptical in shape in the cross-section (relative to the axis of the symmetry of the diffuser). The tip of the spray nozzle feeding the aerosol into the atomising chamber should be located in one of the points located on the opposite site, at the intersection of the semi-major axis with the perimeter of the ellipsis. In this way, the distance between the spray nozzle and the opposite chamber wall is as big as possible which results in the generation of a larger quantity of aerosol.

The design of the diffuser described above allows for a forced aerosol flow from the atomising chamber, through the passage to the lower separation chamber in which the aerosol must penetrate a number of slots created by opposite upper and lower rings and then penetrate to the upper separation chamber and then via the through hole into the atmosphere. Covering this path makes the aerosol lose its momentum and condensate. It is condensed into heavier fractions and larger particles, while smaller and more volatile particles are released to the outside of the diffuser. However, before the aerosol is blown out into the atmosphere, it undergoes condensation once again. The condensate from the upper and lower separation chambers flows down with the help of gravity through the atomising chamber and the discharge channel to the fragrance container, from where it can be sucked in again through the supply channel to the spray nozzle.

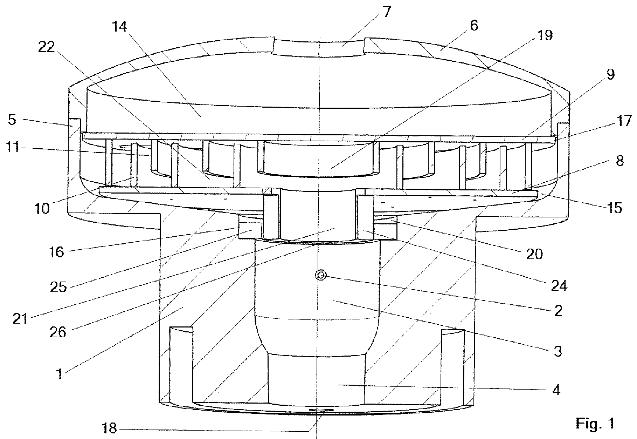

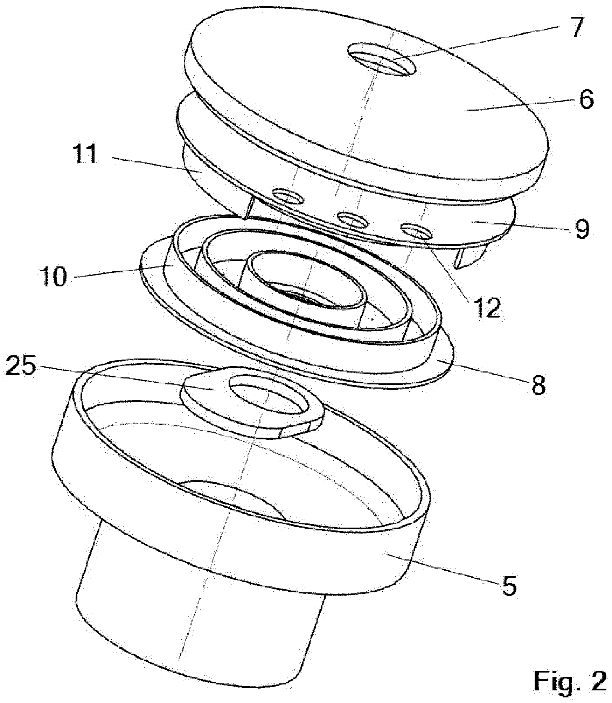

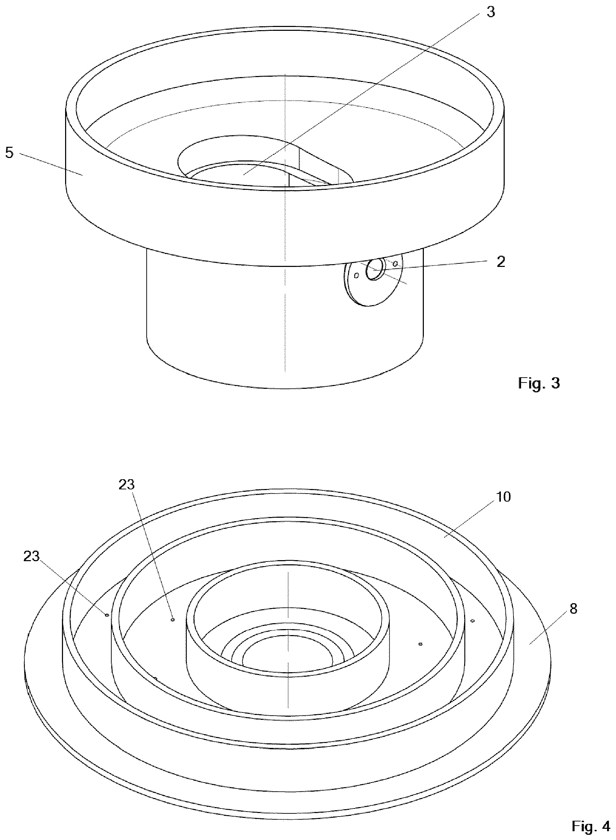

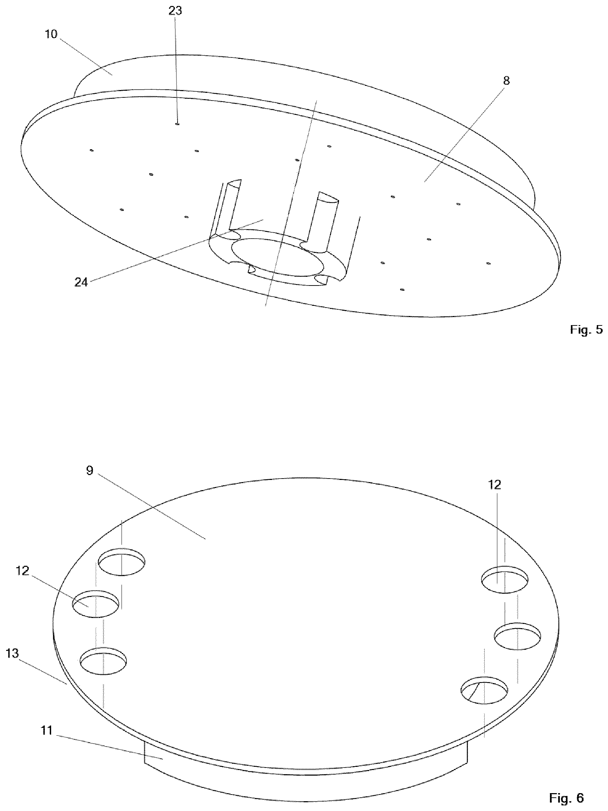

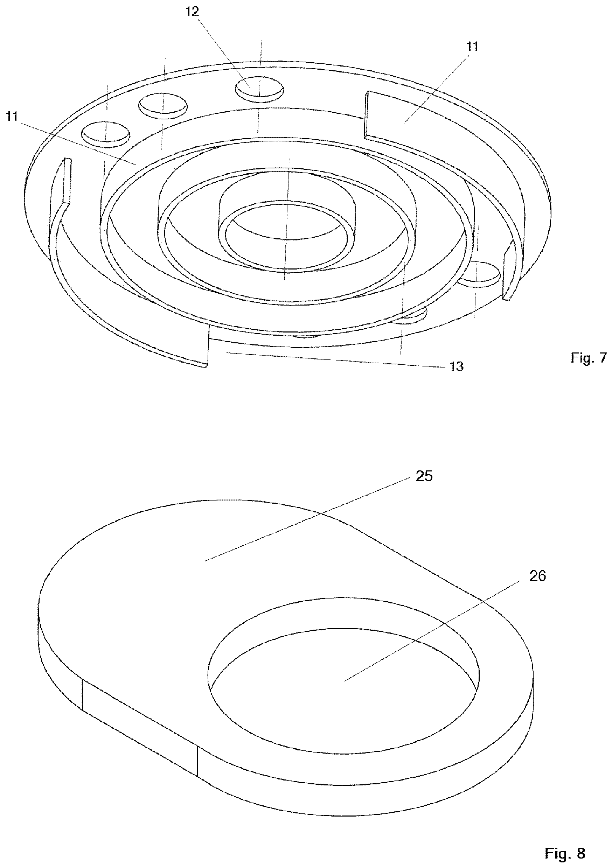

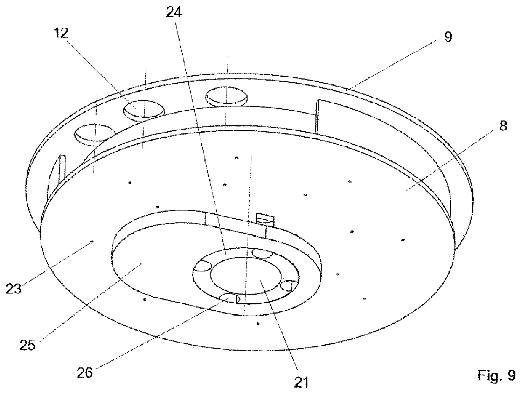

The invention is shown in figures. Fig. 1 presents the diffuser in cross-section. Fig. 2 presents separated parts: the diffuser base, the lower disc, the cover of the atomising chamber, the upper disc and the diffuser cover. Fig. 3 presents the diffuser base in axonometric view, with the oval atomising chamber, widened in the upper part, in the place where the chamber cover is located. Fig. 4 presents the lower disc in the top axonometric view with three lower rings. Fig. 5 presents the lower disc in the bottom axonometric view, with the sleeve and holes of the lower disc. Fig. 6 presents the upper disc in the top axonometric view with six through holes distributed opposite each other and visible upper external ring, on whose path there are gaps visible (circumferential holes). Fig. 7 presents the upper disc in the bottom axonometric view with three full upper rings and the fourth external upper ring with circumferential holes. Fig. 8 presents the cover of the atomising chamber with the cover hole. Fig. 9 presents the folded upper and lower disc and the cover of the atomising chamber with the sleeve of the lower disc put through the hole.

The example of execution presents the invention - a diffuser consisting of a base (1) in the form of a tube connected on the side with the spray nozzle (2) and widened in the upper part to form an elliptical cylinder with an external flange of the base (5).

The spray nozzle (2) penetrates the wall of the diffuser base (1) and is connected to the atomising chamber (3), elliptical in shape, and before the inlet of the spray nozzle (2) to the atomising chamber (3), there is a supply channel (18) connected to the spray nozzle (2) through which the fragrance is drawn from the container. The atomising chamber (3) is located asymmetrically, stretching from the middle of the diffuser base (1) to the edge of the base flange (5). At the bottom of the atomising chamber (3), there is a discharge channel (4) leading to the fragrance container. Whereas in the upper part of the atomising chamber (3), there is a chamber cover (25) with a cover hole (26) located near the axis of symmetry of the diffuser. The sleeve (24), connecting the atomising chamber (3) with the lower separation chamber (19) and centring the position of the lower disc (8), runs through the cover hole (26). The sleeve (24) has the sleeve hole (21) through which the aerosol flows upwards.

Inside the lower separation chamber (19) there is the lower disc (8) with three lower rings (10) in the form of oval flanges with different radii, which are attached to the surface of the lower disc (8) at right angle. In the lower disc (8) between the lower rings (10) there are 16 holes of the lower disc (23) - 0.5 mm each. There are 8 holes of the lower disc (23) between the first and the second lower ring (10), and 8 holes (23) between the second and the third lower ring (10). The holes of the lower disc (23) connect the lower separation chamber (19) with the condensate chamber (20). Through these holes the fragrance condensed in the lower separation chamber (19) flows down into the condensate chamber (20).

Between the perimeter of the lower disc (8) and the base flange (5) there is a middle annular slot (15), 0.7 mm wide, which also connects the lower separation chamber (19) with the condensate chamber (20).

The lower part of the condensate chamber (20) is inclined towards the inside with respect to the lower disc (8) at the angle α 10°. As a result of that, the condensate which penetrates into the condensate chamber (20) flows down towards the chamber cover (25) from where it flows down into the atomising chamber (3) both through the slot between the sleeve (24) and the cover hole (26) and the lower annular slot (16) on the edges of the chamber cover (25).

Above the lower disc (8), there is the upper disc (9) with three upper rings (11) of different sizes. The sizes of the upper rings (11) differ also from the sizes of the lower rings (10), which makes it possible for the upper and lower rings to mesh together (gear alternately). Between the lower rings (10) and the upper disc (9) and between the upper rings (11) and the lower disc (8) there are ring slots (22). These slots are from 1.5 to 5 mm wide.

In the proximity of the outer perimeter of the upper disc (9), there are 3 through holes (12) made in its surface opposite one another, each hole round in shape and with a diameter of 0.9 cm. The through holes (12) connect the lower separation chamber (19) with the upper separation chamber (14). In the largest upper ring (11), positioned nearest to the perimeter of the upper disc (9), there are two circumferential holes (13) which have a form of a gap in the ring perimeter and are located opposite each other, in points where in the upper disc (9) the through holes (12) are made.

Between the edge of the upper disc (9) and the casing of the diffuser there is the upper annular slot (17), which is 0.5 mm wide.

The cover (6) is attached to the base flange (5) of the dispenser from the top. There is the upper separation chamber (14) inside the cover. In the central part of the cover (6), there is a round outlet hole (7) through which the aerosol with the fragrance escapes into the atmosphere.

The experiments carried out proved that due to the described shape of the aroma dispenser diffuser, the problem of contamination of the aroma dispensers emitting aromas dissolved in relatively heavy oils is eliminated. At the same time, it allows to save the aromas dissolved in light oils at the level of up to 20%, which depends on the oil characteristics. The fact is that when using a diffuser constructed in such a way, the composition of the fragrance in the container changes over time, as it thickens and its fractional composition changes. However, in the majority of cases it did not affect the functionality of the dispenser. Similar effect was already noticeable with the use of traditional dispensers, where light fractions evaporated first, causing change in the composition of the unused fragrance.

The invented diffuser is intended substantially to be used with pneumatic dispensers; however, it can be used in other types of dispensers (e.g. those using ultrasonic atomisers or heaters). In such a case, the aerosol flows with the use of vapour pressure energy. However, in the dispensers of this type it is better to use the diffuser with the smaller number of rings in the lower separation chamber, so as not to excessively attenuate the aerosol flow.

## Claims

1. An aroma diffuser consisting of a diffuser base (1) that widens in the upper part, forming a base flange (5), a cover (6) attached to a base flange (5) from the top, an inner atomising chamber (3) connected to a spray nozzle (2), separation chamber (14, 19) where the aerosol undergoes condensation and a round outlet hole (7) located in the central part of the cover (6) through which the aerosol with the fragrance escapes into the atmosphere **characterised in that** the separation chamber (14, 19) is formed by an upper separation chamber (14) inside the cover (6) and a lower separation chamber (19), the lower separation chamber (19) has a lower disc (8) equipped with at least two lower rings (10) of different size mounted substantially concentrically, between which there are holes of the lower disc (8) and above there is an upper disc (9) equipped with at least two upper rings (11) mounted substantially concentrically, each of different size and their sizes differing from those of the lower rings (10), as a result of which the lower rings (10) and upper rings (11) interleave and gear alternately, and between the lower rings (10) and the upper disc (9) and between the upper rings (11) and the lower disc (8) there are ring slots (22) and in addition in the surface of the upper disc (9) there is at least one through hole (12) connecting the lower separation chamber (19) with the upper separation chamber (14) located between the upper disc (9) and the cover (6).

2. The aroma diffuser according to claim 1 **characterised in that** the atomising chamber (3) spreads lengthwise, and has in its top view elliptical or oval shape, and the inlet of the spray nozzle (2) is positioned in an intersection point of a longer axis of symmetry of such oval or ellipse and a wall of the atomising chamber (3).

3. The aroma diffuser according to one of the above-mentioned claims **characterised in that** the holes in the lower disc (23) are substantially round, have a diameter of 0.1 to 3 mm and are distributed between lower rings (10).

4. The aroma diffuser according to claim 3 **characterised in that** in the lower disc there are from 4 to 32 holes of the lower disc (23).

5. The aroma diffuser according to one of the above-mentioned claims **characterised in that** the height of the outer upper ring (11) is greater than the height of other upper rings (11), therefore the lower edge of the outer ring adheres to the surface of the lower disc (8) and in the outer upper ring (11) there is at least one circumferential hole (13).

6. The aroma diffuser according to one of the above-mentioned claims **characterised in that** the lower disc (8) and lower rings (10) and the upper disc (9) and upper rings (11) are round, oval or elliptical in shape.

7. The aroma diffuser according to one of the above-mentioned claims **characterised in that** between the edge of the lower disc (8) and the casing there is a middle annular slot (15) and between the edge of the upper disc (9) and the casing there is the upper annular slot (16) and the width of these slots is from 0.5 to 0.8 mm.

8. The aroma diffuser according to one of the above-mentioned claims **characterised in that** the atomising chamber (3) has at the top the chamber cover (25) equipped with a cover hole (26), into which the sleeve (24) is fitted and between the edge of the cover hole (26) and the sleeve (24) there is a hole or a slot.

9. The aroma diffuser according to one of the above-mentioned claims **characterised in that** below the lower disc (8) there is the condensate chamber (20) whose bottom plane is inclined towards the inside at the angle α 10 to 45°.

## Patentansprüche

1. Ein Aromadiffusor, bestehend aus einem Diffusor-Sockel (1), der sich im oberen Teil erweitert,
und einen Bodenflansch (5) formt, aus einer von oben am Bodenflansch (5) befestigten Abdeckung (6), einer an einer Sprühdüse (2) angeschlossenen inneren Zerstäubungskammer (3), einer Trennkammer (14, 19), in der das Aerosol kondensiert, und aus einer runden Austrittsöffnung (7) im mittleren Teil der Abdeckung (6), durch die das Aerosol mit dem Duftstoff in die Umgebung abgegeben wird, **dadurch gekennzeichnet, dass** die Trennkammer (14, 19) aus einer oberen Trennkammer (14) innerhalb der Abdeckung (6) und aus einer unteren Trennkammer (19) gebildet ist, wobei die untere Trennkammer (19) mit einer unteren Scheibe (8) ausgestattet ist, die mit mindestens zwei hauptsächlich konzentrisch angeordneten unteren Ringen (10) unterschiedlicher Größe ausgestattet ist, zwischen denen sich Öffnungen der unteren Scheibe (8) befinden, und darüber eine obere Scheibe (9) angeordnet ist, die mit mindestens zwei hauptsächlich konzentrisch angeordneten oberen Ringen (11) ausgestattet ist, die jeweils unterschiedlich groß sind und deren Größen sich von denen der unteren Ringe (10) unterscheiden, wodurch die unteren Ringe (10) und die oberen Ringe (11) abwechselnd ineinandergreifen und sich verzahnen, und zwischen den unteren Ringen (10) und der oberen Scheibe (9) sowie zwischen den oberen Ringen (11) und der unteren Scheibe (8) sich Ringschlitze (22) befinden, und in der Oberfläche der oberen Scheibe (9) sich mindestens ein Durchgang (12) befindet, der die untere Trennkammer (19) mit der oberen Trennkammer (14) verbindet, die zwischen der oberen Scheibe (9) und der Abdeckung (6) angeordnet ist.

2. Ein Aromadiffusor nach Anspruch 1, **dadurch gekennzeichnet, dass** die Zerstäubungskammer (3) sich in Längsrichtung erstreckt und von oben gesehen, eine elliptische oder ovale Form aufweist, und dass der Einlass der Sprühdüse (2) an einem Schnittpunkt der längeren Symmetrieachse dieses Ovals oder dieser Ellipse und einer Wand der Zerstäubungskammer (3) angeordnet ist.

3. Ein Aromadiffusor nach einem der vorgenannten Ansprüche, **dadurch gekennzeichnet, dass** die Öffnungen in der unteren Scheibe (23) hauptsächlich rund sind, einen Durchmesser von 0,1 bis 3 mm aufweisen und zwischen den unteren Ringen (10) verteilt sind.

4. Ein Aromadiffusor nach Anspruch 3, **dadurch gekennzeichnet, dass** die untere Scheibe (23) 4 bis 32 Öffnungen aufweist.

5. Ein Aromadiffusor nach einem der vorgenannten Ansprüche, **dadurch gekennzeichnet, dass** die Höhe des äußeren oberen Rings (11) größer ist als die Höhe der sonstigen oberen Ringe (11), wodurch die Unterkante des äußeren Rings an der Oberfläche der unteren Scheibe (8) anliegt, und dass der äußere obere Ring (11) mindestens eine umlaufende Öffnung (13) aufweist.

6. Ein Aromadiffusor nach einem der vorgenannten Ansprüche, **dadurch gekennzeichnet, dass** die untere Scheibe (8) und die unteren Ringe (10) sowie die obere Scheibe (9) und die oberen Ringe (11) eine runde, ovale oder elliptische Form aufweisen.

7. Ein Aromadiffusor nach einem der vorgenannten Ansprüche, **dadurch gekennzeichnet, dass** ein mittlerer ringförmiger Schlitz (15) sich zwischen dem Rand der unteren Scheibe (8) und dem Gehäuse und ein oberer ringförmiger Schlitz (16) sich zwischen dem Rand der oberen Scheibe (9) und dem Gehäuse befindet und die Breite dieser Schlitze zwischen 0,5 und 0,8 mm beträgt.

8. Ein Aromadiffusor nach einem der vorgenannten Ansprüche, **dadurch gekennzeichnet, dass** die Zerstäubungskammer (3) oben mit einer Kammerabdeckung (25) ausgestattet ist, die eine Öffnung (26) aufweist, in die eine Hülse (24) eingeführt ist, und dass zwischen dem Rand der Öffnung (26) und der Hülse (24) eine Öffnung oder ein Schlitz vorhanden ist.

9. Ein Aromadiffusor nach einem der vorgenannten Ansprüche, **dadurch gekennzeichnet, dass** sich unterhalb der unteren Scheibe (8) eine Kondensatkammer (20) befindet, deren Bodenfläche ein Gefälle von 10 bis 45° nach innen aufweist.

## Revendications

1. Diffuseur d'arôme comprenant une base de diffuseur (1) s'élargissant dans sa partie supérieure
pour former une collerette (5), un couvercle (6) fixé sur ladite collerette (5), une chambre interne d'atomisation (3) reliée à une buse de pulvérisation (2), une chambre de séparation (14, 19) dans laquelle l'aérosol subit une condensation, ainsi qu'un orifice de sortie (7) disposé dans la partie centrale du couvercle (6) pour permettre l'évacuation de l'aérosol parfumé, **caractérisé en ce que** la chambre de séparation (14, 19) comprend une chambre de séparation supérieure (14) disposée à l'intérieur du couvercle (6) et une chambre de séparation inférieure (19), **en ce que** la chambre de séparation inférieure (19) comprend un disque inférieur (8) muni d'au moins deux anneaux inférieurs (10) de dimensions différentes, disposés sensiblement de manière concentrique, des orifices étant ménagés entre lesdits anneaux, et **en ce que** le disque supérieur (9) disposé au-dessus du disque inférieur (8) est muni d'au moins deux anneaux supérieurs (11), disposés sensiblement de manière concentrique et de dimensions différentes de celles des anneaux inférieurs (10), de sorte que les anneaux inférieurs (10) et supérieurs (11) s'entrelacent de manière alternée, et **en ce que** des fentes annulaires (22) sont formées entre les anneaux inférieurs (10) et le disque supérieur (9), ainsi qu'entre les anneaux supérieurs (11) et le disque inférieur (8), et **en ce que** le disque supérieur (9) comporte au moins un orifice traversant (12) reliant la chambre de séparation inférieure (19) à la chambre de séparation supérieure (14) située entre le disque supérieur (9) et le couvercle (6).

2. Diffuseur d'arôme selon la revendication 1, **caractérisé en ce que** la chambre d'atomisation (3) s'étend longitudinalement et présente, en vue de dessus, une forme elliptique ou ovale, et **en ce que** l'entrée de la buse de pulvérisation (2) est disposée au point d'intersection de l'axe de symétrie longitudinal de ladite forme elliptique ou ovale avec une paroi de la chambre d'atomisation (3).

3. Diffuseur d'arôme selon l'une quelconque des revendications précédentes, **caractérisé en ce que** les orifices du disque inférieur (23) sont sensiblement circulaires, présentent un diamètre compris entre 0,1 et 3 mm et sont répartis entre les anneaux inférieurs (10).

4. Diffuseur d'arôme selon la revendication 3, **caractérisé en ce que** le disque inférieur (23) comporte entre 4 et 32 orifices.

5. Diffuseur d'arôme selon l'une quelconque des revendications précédentes, **caractérisé en ce que** la hauteur de l'anneau supérieur extérieur (11) est supérieure à celle des autres anneaux supérieurs (11), de sorte que le bord inférieur dudit anneau extérieur est en appui sur la surface du disque inférieur (8), et **en ce que** ledit anneau supérieur extérieur (11) comporte au moins un orifice périphérique (13).

6. Diffuseur d'arôme selon l'une quelconque des revendications précédentes, **caractérisé en ce que** le disque inférieur (8) et les anneaux inférieurs (10), ainsi que le disque supérieur (9) et les anneaux supérieurs (11), présentent une forme circulaire, ovale ou elliptique.

7. Diffuseur d'arôme selon l'une quelconque des revendications précédentes, **caractérisé en ce qu'**un espace annulaire intermédiaire (15) est ménagé entre le bord du disque inférieur (8) et le boîtier, et un espace annulaire supérieur (16) est ménagé entre le bord du disque supérieur (9) et le boîtier, la largeur desdits espaces étant comprise entre 0,5 et 0,8 mm.

8. Diffuseur d'arôme selon l'une quelconque des revendications précédentes, **caractérisé en ce que** la chambre d'atomisation (3) comporte, dans sa partie supérieure, un couvercle de chambre (25) muni d'un orifice (26), dans lequel est insérée une douille (24), et **en ce qu'**un jeu, sous forme d'un orifice ou d'une fente, est ménagé entre le bord dudit orifice (26) et ladite douille (24).

9. Diffuseur d'arôme selon l'une quelconque des revendications précédentes, **caractérisé en ce qu'**au-dessous du disque inférieur (8) est disposée une chambre de condensation (20) dont le fond est incliné vers l'intérieur selon un angle compris entre 10° et 45°.
